# EUROPEAN PATENT SPECIFICATION

(11) **EP 0 624 576 B1**
(45) Date of publication and mention of the grant of the patent: **25.10.2000**
(21) Application number: 94107240.7
(22) Date of filing: 09.05.1994
(51) Int. Cl.: C07D 251/70, C08G 12/32

(54) **Monomeric aminoplasts crosslinking agents**
Monomere Aminoplaste als Vernetzungsmittel
Aminoplastes monomères comme agents de réticulation

(30) Priority: 10.05.1993 US 58085; 13.05.1993 US 60135
(43) Date of publication of application: 17.11.1994
(73) Proprietor: CYTEC TECHNOLOGY CORP., West Paterson New Jersey 07424 (US)
(72) Inventor: Szita, Jeno George, Norwalk, Connecticut 06850 (US); Lees, Robert Gerald, Stamford, Connecticut 06905 (US)
(74) Representative: DIEHL GLAESER HILTL & PARTNER

(56) References cited:
- EP-A- 0 203 879
- EP-A- 0 366 884
- US-A- 4 230 740
- US-A- 5 294 671

## Description

This invention relates to monomeric aminoplasts, process for preparing and use of same as crosslinking agents.

To effectively crosslink with a variety of widely available difunctional materials such as diols, dicarboxylic acids, dimercaptans, and diamides, a monomeric crosslinking agent is required to have a functionality effective for crosslinking of at least two. In many cases, however, even trifunctional aminoplast crosslinking agents do not give rise to sufficient crosslinking density in cured films or objects due to incomplete reaction of the functional groups, and as a result, cured films with inferior physical and resistance properties are obtained.

The problem of insufficient crosslinking density may be overcome by using a higher functional aminoplast crosslinker such as hexamethoxymethyl melamine. In these cases, however, the films obtained sometimes have low flexibility due to the somewhat rigid network produced in the films upon cure.

US-A-4,230,740 discloses photopolymer compositions comprising a polythiol; a photoinitiator and a polyene, wherein the polyene is a hexamethylolmelamine derivative comprising at least three polymerizable vinyl substituents.

EP-A-0,366,884 discloses coating and adhesive compositions comprising vinyl-terminated carbamylmethylated melamines and vinyl-terminated polyurethane/polyamide polymers.

EP-A-0,203,879 discloses the preparation of polymerizable monomers prepared from 1-(1-isocynato-1-methylethyl)-4-(1-methylethenyl)benzene and compounds containing a Zerewitinoff active hydrogen atom.

The above-identified problems of insufficient crosslinking of the low functionality crosslinkers and the low flexibility of the highly functional crosslinders are both overcome by using typically tetrafunctional guanamine-derived aminoplast crosslinking agents. However, guanamine-derived crosslinkers are more difficult and more costly to prepare than melamine-derived crosslinkers. Furthermore, some guanamine crosslinkers such as N,N,N',N'-tetraalkoxymethylbenzoguanamines have insufficient resistance properties and have inferior stability towards the degradative action of ultraviolet light.

It is the object of this invention to obtain melamine-derived, substantially monomeric aminoplasts useful as crosslinking agents having olefinic functionality which are capable of producing, upon cure, films which have good acid resistance properties, environmental etch resistance, and a good balance of hardness and flexibility.

This object is solved by the composition according to independent claim 1, the process of producing same according to independent claim 7, the process for preparing an alkoxymethylated aminoplast according to independent claim 8, the curable composition according to independent claim 10, the method of coating a substrate according to independent claim 15, and the cross linked film or object according to independent claim 17. Further advantageous features, aspects and details of the invention are evident from the dependent claims, the description and the examples. The claims are to be understood as a first non-limiting approach to define the invention in general terms.

This invention relates to a novel, highly functional substantially monomeric aminoplast crosslinking agent and an intermediate used in the production thereof.

This invention also relates to a process for preparing said crosslinking agent.

This invention also relates to a curable composition containing said highly functional substantially monomeric aminoplast crosslinking agent.

This invention also relates to an improved method of coating using the curable composition of the invention.

Finally, this invention relates to a cured film or object prepared by the improved method of the invention.

The cross linkers may be prepared from 2,4,6-triamino-1,3,5-triazine, hereinafter "melamine", and isopropenyl-alpha, alpha-dimethylbenzyl isocyanate, hereinafter "TMI", by a monoaddition reaction followed by methylolation and etherification.

This invention relates to meta-, para-, or a mixture of meta- and para-isopropenyl-alpha, alpha-dimethylbenzyl isocyanate adducts represented by the formula: 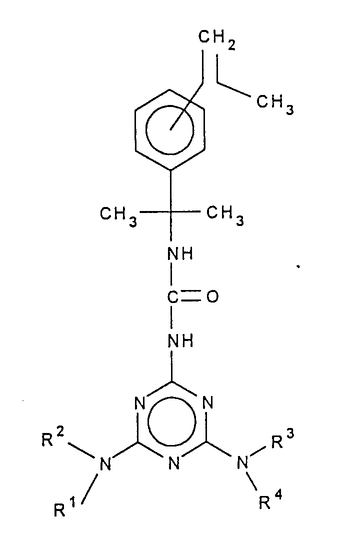 wherein each of R¹, R², R³, and R⁴ is independently selected from the group consisting of hydrogen, hydroxymethyl, an alkoxymethyl.

The preferred isomer of the aminoplast is the meta- isomer having at least one of the R¹, R², R³, and R⁴ groups selected independently from the group consisting of hydroxymethyl, alkoxymethyl of 1 to 6 carbon atoms, and mixtures thereof.

The most preferred aminoplasts are compositions of matter represented by the formula: 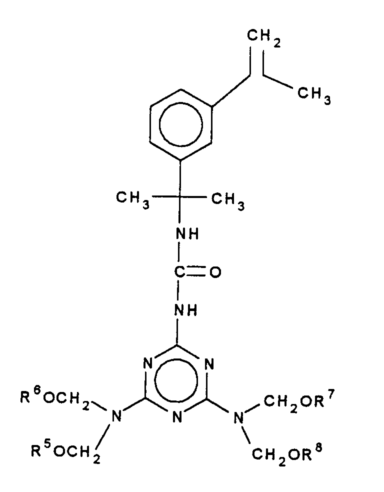 wherein each of R⁵, R⁶, R⁷, and R⁸ is independently selected from the group consisting of hydrogen, methyl, ethyl, 1-propyl, 2-propyl, 1-butyl, 2-butyl, iso-butyl, 1-pentyl, 1-hexyl and cyclohexyl groups, and mixtures thereof.

An example of the aminoplasts containing mixed alkoxy crosslinkably reactive groups is the particularly preferred composition of matter represented by the formula: 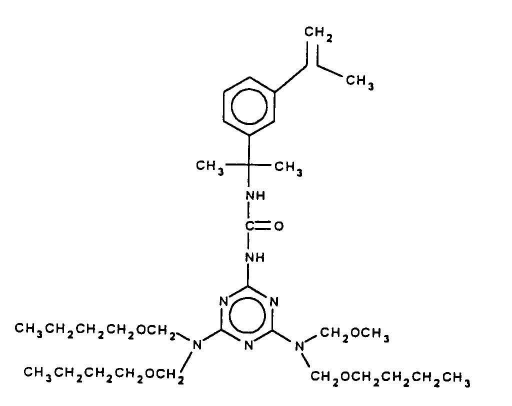 wherein the ratio of normal butyl to methyl groups is 3:1.

The novel, substantially monomeric aminoplast crosslinking agents of the invention may be prepared by a process in which a 1:1 adduct of melamine with an isocyanate is chemically modified to contain hydroxy-methyl, alkoxymethyl or both groups. The isocyanate which forms the adduct is meta-isopropenyl alpha,alpha-dimethylbenzyl isocyanate(hereinafter m-TMI), available as TMI® (meta) Unsaturated Aliphatic Isocyanate, a product of Cytec Industries Inc., West Paterson, New Jersey, or para-isopropenyl-alpha, alpha-dimethyl-benzyl isocyanate (hereinafter p-TMI), or a mixture of the meta- and para- isomers. The para-isomer may be prepared by procedures described in U.S. Patent Nos. 3,290,350; 4,130,577; 4,377,530; or 4,439,616.

The reaction steps for preparing the novel tetra functional cross linkers are illustrated below for the meta-isomer:

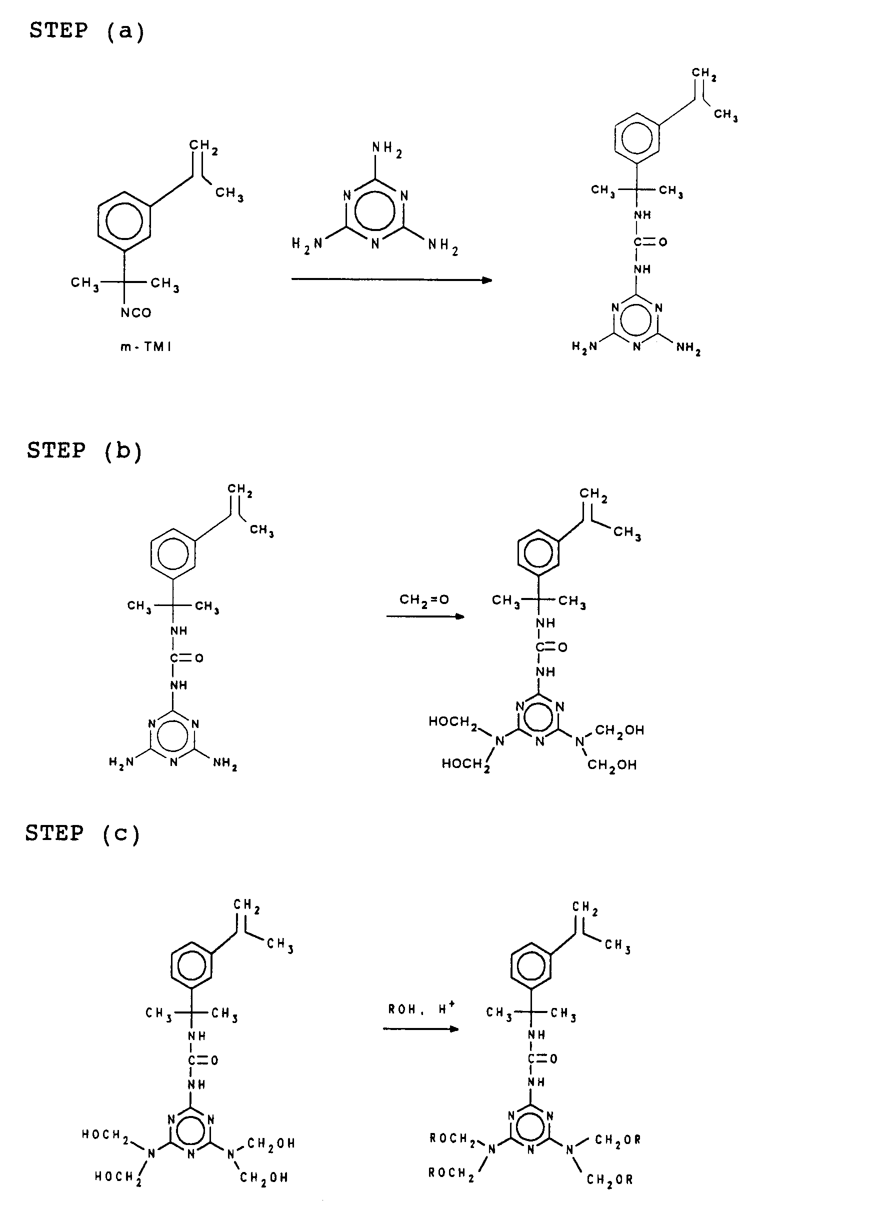

The novel process for preparing the aminoplast of the invention comprises:
(a) contacting melamine with isopropenyl-alpha,alpha-dimethylbenzyl isocyanate in a solvent characterized by a high dielectric constant, at a temperature and for a length of time sufficient to produce a 1:1 adduct,
(b) contacting said 1:1 adduct of step (a) with 2 to 20 moles of formaldehyde per mole of adduct to produce a hydroxymethylated adduct, and
(c) contacting said hydroxymethylated adduct of step (b) with 2 to 30 moles of an alcohol per mole of hydroxymethylated adduct at an acidic pH to produce an alkoxymethylated aminoplast.

In step (a), the monoadduct of TMI and melamine is prepared using a 1:1 molar ratio of TMI to melamine, normally preferred on the basis of reaction stoichiometry. However, the adduct-forming reaction may be carried out at any ratio. For example, if a TMI to melamine molar ratio of 0.5:1 is used, there will remain a large excess of unreacted melamine which may be separated from the product, which product is necessarily a 1:1 adduct. If, on the other hand, excess quantities of TMI such as a 5:1 molar excess are used, only the monoaddition product is obtained under the process conditions of this invention. The unreacted TMI in this case may be easily removed by precipitation of the product.

The preferred solvents in step (a) are aprotic solvents having relatively high boiling points, high dipole moments and high dielectric constants for facilitating the dissolution of the sparingly soluble melamine at the reaction temperature and for allowing the product to crystallize at ambient temperatures. The preferred solvent is dimethylsulfoxide, however, aprotic solvents such as N,N-dimethylformamide, N,N-dimethylacetamide, N-methyl pyrrolidone, sulfolane, hexamethylphosphorus triamide (HMPT), hexamethylphosphoramide (HMPA), and mixtures thereof may also be used.

Any solvent to reactant ratio may be used to carry out step (a) of the process of the invention. The preferred range of the solvent to reactant ratio is from about 0.33:1 to about 10:1. Most preferably, the ratio is 2:1.

The preferred temperature for carrying out the monoaddition reaction of step (a) is in the range of from about 80 °C to about 150 °C. At temperatures lower than 80 °C, the reaction proceeds at a very slow rate. At temperatures higher than 150 °C, side reactions, including decomposition of the solvent and TMI reactant, may become significant. A temperature in the range of 100 °C to 120 °C is most convenient to prepare the 1:1 adduct.

The preferred time for carrying out the monoaddition reaction of step (a) is in the range of from about 12 minutes to about 28 hours.

After the monoaddition reaction of step (a), the 1:1 adduct is isolated by cooling the reaction mixture and filtering the precipitated product. The product may be further purified by washing with an organic solvent capable of dissolving the solvent used in the process of the invention. An example of a solvent usable for this purpose is tetrahydrofuran.

In step (b) of the process, the adduct is hydroxymethylated (or methylolated) with 1 to 20 moles of formaldehyde per mole of the adduct, typically in water or an alcohol, or in a mixture of water and an alcohol such as normal butanol (n-butanol).

In step (c), the hydroxy groups in the methylolated 1:1 adduct are etherified with an alcohol under acidic conditions, typically at a pH range from 0.5 to 6.0, and preferably from about 2 to 4. The alcohol used to etherify the methylolated 1:1 adduct is usually used in a large excess to ensure a high degree of etherification and to prevent self-crosslinking of the product. Therefore, the alcohol used for etherification typically is the reaction solvent.

When mixed alkoxymethylated aminoplasts are desirable, a mixture of alcohols may be used both as reactant and as solvent.

### Curable Composition

The novel aminoplasts of the invention may be used as crosslinking agents in curable compositions to produce, upon curing, crosslinked films or objects useful in coatings, adhesives, conventional moldings, reactive injection moldings, composites, laminates, binders, and others.

The curable composition comprises:
(i) an aminoplast crosslinking agent represented by the formula: 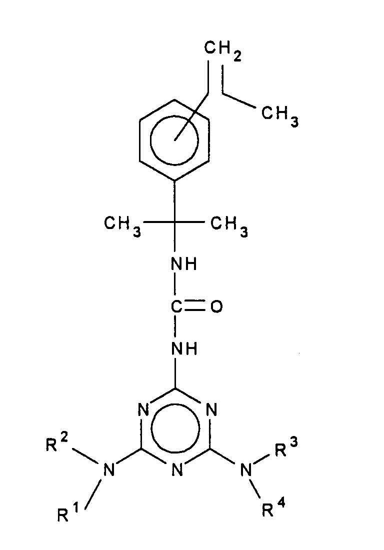 wherein the point of attachment of the isopropenyl group is meta-, or para-, or a mixture thereof, and wherein each of R¹, R², R³, and R⁴ is independently selected from the group consisting of hydrogen, hydroxymethyl, an alkoxymethyl of preferably 1 to 6 carbon atoms, an aminoplast-containing group derived from condensation thereof, and mixtures of any of the preceding groups. Furthermore, preferred is that at least one of the R¹, R², R³, and R⁴ groups are independently selected from the group consisting of hydroxymethyl, an alkoxymethyl of 1 to 6 carbon atoms, and mixtures thereof; and
(ii) a polyfunctional active hydrogen-containing material.

### Aminoplast Crosslinking Agents

The aminoplast crosslinking agents usable in the curable compositions are the novel aminoplasts of the invention having at least two crosslinkably reactive functions, preferably at least one of which is selected from the group consisting of hydroxymethyl, alkoxymethyl, and a mixture thereof.

### Polyfunctional Materials

The polyfunctional materials usable in the invention are polyfunctional active hydrogen-containing materials.

Suitable polyfunctional materials may be polymercaptans, polycarboxylic acids, polyamides, epoxy or urethane prepolymers, alkyds, and polyols such as acrylic resins containing pendant or terminal hydroxyl functionalities, polyester resins with pendant or terminal hydroxyl functionalities and polyhydric alcohols. These are described in greater detail below.

The polyfunctional materials and resins used in the compositions of the invention preferably have a molecular weight of from about 60 to about 50,000 and comprise at least one class of an active hydrogen functionality selected from the group consisting of hydroxy, carboxy, amido, mercapto, and a group convertible thereto.

The hydroxyfunctional polyfunctional materials used in formulating the curable compositions of this invention preferably are resins that have molecular weights in the range of from about 500 to about 50,000, and hydroxyl group equivalent weights of from about 200 to about 4,000.

An example of a suitable polyfunctional polyester resin usable in the curable compositions of this invention is OXYESTER® Z 1439 Branched Polyester Resin, a product of Chemische Werke Hüls AG, Germany having the following physical and chemical properties:

| | |
|---|---|
| Hydroxyl Content (% by weight) | 2 |
| Hydroxyl Number | 65 |
| Equivalent Weight | 863 |
| Solids Content (% by weight) | 50 |

Another example of a suitable polyfunctional polyester resin particularly suited for use in coil coatings is CYPLEX® 1531 modified Polyester Resin, a product of Cytec Industries Inc., West Paterson, New Jersey, having the following physical and chemical properties:

| | | |
|---|---|---|
| Solids | (Weight %) | 60 |
| | (Volume %) | 52.9 |
| Color (Gardner 1963) | | 6 (max.) |
| Viscosity (Gardner-Holt, 25 °C) | | Y-Z₂ |
| Hydroxyl Number (solids) | | 30 |
| Equivalent Weight (solids) | | 1,870 |
| Molecular Weight, approximate | | 4,000 |
| Acid number (solids) | | 10 (max.) |
| Solvesso 150 Aromatic Hydrocarbon Solvent (%) (a product of Humble Oil and Refining Company) | | 40 |

Another example of a suitable polyfunctional resin for coil coating is CYPLEX® 1538 Modified Polyester Resin, a product of Cytec Industries Inc., West Paterson, New Jersey, having the following properties:

| | | |
|---|---|---|
| Solids | (Weight %) | 65 |
| | (Volume) | 58 |
| Color (Gardner 1963) | | 6 (max.) |
| Viscosity (Gardner-Holt, 25°C) | | Z₁ - Z₃ |
| Hydroxyl Number (Solids) | | 40 |
| Equivalent Weight (Solids) | | 1400 |
| Molecular Weight, approximate | | 2800 |
| Acid Number (Solids) | | 10 (max.) |
| Solvesso 150 Aromatic Hydrocarbon Solvent (%) | | 35 |

Another example of a suitable polyfunctional resin particularly suited to coil coatings is CYPLEX® 1546 Oil-Free Polyester Resin, a product of Cytec Industries Inc., West Paterson, New Jersey, having the following properties:

| | |
|---|---|
| Non-Volatiles (Weight %) | 70 ± 2 |
| Color (Gardner 1963, max.) | 4 |
| Viscosity (Gardner-Holt, 25 °C) | Z₁ - Z₃ |
| Acid Number (resin solids, max) | 10 |
| Hydroxyl Number (resin solids) | 35 - 40 |
| Equivalent weight | 1,400 - 1,600 |

An example of a suitable acrylic resin for non-coil coating applications is JONCRYL® 500 Acrylic Resin, a product of S.C. Johnson and Son, Inc., Racine, Wisconsin, having the following properties:

| | |
|---|---|
| Solids Content (Weight %) | 80 |
| Viscosity at Room Temperature mPa·s (Centipoise) | 4,000 (4000) |
| Hydroxyl Number (based on solids) | 140 |
| Equivalent Weight (based on solids) | 400 |
| Molecular Weight (Mn)* | 1,300 |
| Polydispersity (Mw/Mn)** | 1.7 |

| | |
|---|---|
| *Mn = Number Average Molecular Weight | |
| **Mw = Weight Average Molecular Weight | |

ARAKOTE® 3109 Hydroxy-Terminated Polyester Resin, a product of Ciba-Geigy Corporation, Hawthorne, New York, is an example of a solid polyester resin particularly suitable to powder coating, and has the following physical and chemical properties:

| | |
|---|---|
| Hydroxyl Number | 27 - 32 |
| Equivalent Weight | 1,900 |
| Tg (Glass Transition, °C) | 66 |
| ICI Viscosity at 200°C mPa·s (Poise) | 4000 (40) |
| Appearance | Colorless Solid |

JONCRYL® SCX-800 A Acrylic Resin and JONCRYL® SCX-800 B Acrylic Resin, products of S.C. Johnson and Son, Inc., examples of solid acrylic resins, also are suitable for powder coatings, and have the following physical and chemical properties:

| | | |
|---|---|---|
| | SCX-800A | SCX-800B |
| Non-Volatiles (Weight %) | 98 | 97 |
| Hydroxyl Number | 43 | 40 |
| Equivalent Weight | 1300 | 1402 |
| Acid Value (mg KOH/g) | 15 | 15 - 20 |
| Tg (Glass Transition, °C) | 43 | 43 |
| Softening Point (°C) | 100 | 107 |
| ICI Viscosity at 200°C mPa·s | 2500 | 4500 - 5000 |
| (Poise) | (25) | (45 - 50) |

In addition to the examples cited above, a variety of commercial polyester resins may be used as the polyfunctional ingredient of the invention, provided such resins have suitable chemical and physical properties similar to those set forth above for ingredient (ii).

Optionally, the curable compositions of the invention may further comprise a cure catalyst to accelerate the curing process at a given temperature or to reduce the cure temperature at a given cure time.

The catalyst, if present, is typically an acid selected from the group consisting of sulfonic, carboxylic, phosphoric, sulfuric, and nitric acids. The preferred acid catalysts are sulfonic acids, including benzenesulfonic acid, paratoluenesulfonic acid, naphthalenesulfonic acid, dinonylnaphthalenesulfonic acid, dodecylbenzenesulfonic acid, methanesulfonic acid, and mixtures thereof.

### Other Ingredients

The curable compositions of the invention may optionally contain a liquid medium, which liquid medium may be used to aid the uniform application and transport of the curable composition. Any or all of the ingredients of the composition may be contacted with the liquid medium. Moreover, the liquid medium may permit formation of a dispersion, suspension, emulsion, invert emulsion, or solution of the curable composition ingredients, including other optional ingredients.

Particularly preferred is a liquid medium which is a solvent or a diluent for the curable composition ingredients (i) and (ii). The preferred solvent or diluent is selected from the group consisting of water, alcohols, ketones, ethers, esters, aliphatic hydrocarbons, aromatic hydrocarbons, halogenated hydrocarbons, and mixtures thereof.

Other optional ingredients include fillers, pigments, flow control agents, anticratering additives, antioxidants, stabilizing alcohols, ultraviolet light stabilizers, plasticizers, pigment wetting additives, levelling additives, mar-proofers, mold release agents, and corrosion inhibitors.

### Ratio Of The Ingredients

The weight ratio of the crosslinker (i) to polyfunctional material (ii) is preferably from about 3:1 to about 1:40 and more preferably 1:1 to 1:5. The weight percent of the crosslinker (i) in the curable composition may be from about 2.5 to about 75.

The weight ratio of the catalyst, when present, to the crosslinker (i) in the curable composition is from about 1:4 to about 1:1,000 and the weight percent of catalyst in the curable composition is from about 0.01 to about 5.

The weight percent of the optional liquid medium ranges from zero to about 80 and the weight ratio of the liquid medium to the total weight of the ingredients (i) and (ii) of the composition ranges from about 0.001 to about 4.

### Improved Method Of Coating

The curable compositions may be used in the improved method of the invention to prepare coatings such as solution coatings, emulsion coatings, powder coatings, coil coatings, electrodeposition coatings, and the like. They may also be used as laminating resins, adhesives or molding compounds.

This invention, therefore, is an improved method for coating of the type having the steps of (I) contacting a substrate with a curable composition containing a crosslinking agent and a polyfunctional active hydrogen-containing material, and (II) thereafter curing, wherein the improvement comprises:
(A) contacting said substrate with a curable composition comprising:
   (i) an aminoplast crosslinking agent represented by the formula: 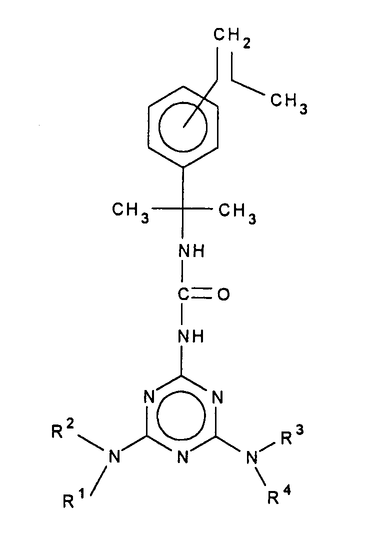 wherein the point of attachment of the isopropenyl group is meta- or para-, or a mixture thereof, and wherein each of R¹, R², R³, and R⁴ is independently selected from the group consisting of hydrogen, hydroxymethyl, an alkoxymethyl of preferably 1 to 6 carbon atoms, an aminoplast-containing group derived from condensation thereof, and mixtures of any of the preceding groups, wherein preferably by at least one of the R¹, R², R³, and R⁴ groups are independently selected from the group consisting of hydroxymethyl, alkoxymethyl of 1 to 6 carbon atoms, and mixtures thereof, and
   (ii) a polyfunctional active hydrogen-containing material, and
(B) thereafter heat-curing said curable composition.

The curable compositions usable in the improved method are the novel curable compositions of the invention described hereinabove containing the novel aminoplast crosslinking agents of the invention having at least two crosslinkable reactive functions at least one of which is selected from the group consisting of hydroxymethyl, alkoxymethyl, and mixtures thereof.

The curable composition may be applied onto a substrate by spraying, padding, brushing, roller-coating, curtaincoating, flowcoating, electrocoating, dipping, or electrostatic spraying. The applied curable composition is thereafter cured, typically, at a temperature in the range of from about 80 °C to about 160 °C within a period of, typically, 5 minutes to 1 hour to produce crosslinked films or objects.

### EXAMPLE 1

### PREPARATION OF m-TMI/MELAMINE 1:1 ADDUCT

In a 2 liter three neck glass reactor equipped with agitator, thermometer, reflux condenser, nitrogen inlet and dropping funnel, 252 g (2 moles) of melamine was dispersed in 800 ml of DMSO (Dimethylsulfoxide) under vigorous agitation. A solution of 423 g (2.1 moles) of m-TMI in 200 ml DMSO was added to the slurry at 110-113 °C in 3.5 hours. The reaction temperature was maintained for an additional hour. To maintain efficient agitation, the thickening slurry was diluted several times during the reaction with a total of 150 ml of DMSO. After cooling to ambient temperature, the reaction mixture was filtered, the white solid was rinsed on a filter with THF (tetrahydrofuran) and reslurried in 800 ml of THF. After agitation at 64 °C for 80 minutes, the product was filtered, rinsed on the filter with THF and dried first in a hot air circulation oven at 60 °C overnight (16 hours), then in a vacuum oven (full pump vacuum) at 90-100 °C for 4 hours. The yield of the white solid product was 665 g. From the first filtrate 85 g and from the second (wash) 73 g of white solids were recovered. Infrared (IR) and Thermal Gravimetric Analysis (TGA) indicated that these products still contained 15-20% of DMSO.

The main product was characterized by NMR (Nuclear Magnetic Resonance), IR and thermal analysis. It is practically insoluble in most organic solvents, sparingly soluble in aprotic solvents such as DMSO, N-methyl-pyrrolidone, DMF (dimethylformamide), etc. Both carbon and proton NMR, as well as the IR spectra were consistent with a 1:1 adduct structure. TGA indicated about a 20% weight loss in the range of 100-125 °C (DMSO). Major weight loss is observed above 245 °C. Major thermal event by DSC (differential scanning calorimetry) is an endotherm occurring in the temperature range in which the major weight loss is observed by TGA (extrap. onset: 223 °C). Liquidification is observed by TM (Thermal Microscopy) in the same range (onset 232 °C) and "boiling" started at 248 °C.

### EXAMPLE 2

### PREPARATION OF MIXED ALKOXYMETHYLATED MONOMERIC AMINOPLAST CROSSLINKING AGENT

A suitable reactor equipped with stirrer, reflux condenser and thermometer was charged with 120 g of butyl formcel (butyl formcel comprises formaldehyde (40%), normal butanol (53%), and water (7%) by weight) and the pH adjusted with 20% caustic solution to 10.1. Then 80 g of the product of Example 1 was added and the temperature of the slurry raised to 85 °C; after eight minutes a clear solution was formed. The temperature was maintained at 85-90 °C for an additional 30 minutes, then 84 g of n-butanol was added. At 65 °C, the pH was adjusted to 2.5 by addition of 0.5 ml of 70% nitric acid and the temperature was maintained for 20 minutes. During the following 35 minutes, 55 ml of distillate was removed at 65-68 °C and about 27 kilo pascals (200 mm Hg). The distillate was replaced by adding portionwise to the reactor the same amount of n-butanol. The reaction mixture was cooled to 35 °C and 1.25 ml 20% caustic was added to adjust the pH to 9.6. The volatiles were stripped to 96°C/about 13 kilo pascals (100 mm Hg) and 115 g of distillate was collected. At 55 °C, 96 g of methanol was charged followed by 0.5 ml of 70% nitric acid at 40 °C (pH=2.1), and the temperature was maintained at 40-43 °C for 40 minutes. After adjusting the pH to 9.5 with 1.2 ml of 20% caustic solution, 112 g distillate was removed at 12 kilo pascals (90 mm Hg) up to 95 °C. The 120 g of the colorless, moderately viscous resin obtained was diluted with 17 g of toluene and filtered at 80 °C under about 276 kilo pascals (approximately 2,000 mm Hg pressure) of nitrogen gas, to give a clear, colorless resin, the novel crosslinking agent of the invention, having the following characteristics:
- HPSEC:: 81.7% Monomer (High Performance Size Exclusion Chromatography Areas)
14.8% Dimer (Areas)
3.5% Trimer (Areas)
- NMR:: CH₃/CH₂ = 0.22 (ratio)
nBu/CH₂ = 0.67 (ratio)
CH₂/Adduct= 3.3 (ratio)
- FREE CH₂O:: 0.55% (by weight)
- METHYLOL:: 2.48% (by weight)
- SOLIDS:: Pan = 89.2% (by weight)
Foil = 95.2% (by weight)
- CH₂O/ADDUCT:: 3.12 (Based on bound formaldehyde and nitrogen analysis)

### EXAMPLE 3

The procedure of Example 2 was repeated with the exception that methyl formcel (methyl formcel comprises formaldehyde (55%), methanol (35%) and water (10%) by weight) was used instead of butyl formcel, and the n-butanol was replaced with methanol. The product was a methoxymethylated analog of the product of Example 2. The methoxymethylated product is another example of the crosslinking agents of the invention and has the following characteristics:
- NMR:: CH₃/CH₂ = 0.96 (ratio)
CH₂O/Adduct = 1.7
- FREE: CH₂O = 0.97% (by weight)
CH₂OH = 1.93% (by weight)
- RESIDUAL DMSO: = 3% (by weight)
- HPSEC:: 73.3% Monomer (High Performance Size Exclusion Chromatography Areas)
16.3% Dimer (Areas)
2.9% Trimer (Areas)
6.9% Higher Oligomers (Areas)
- FOIL SOLIDS:: 84.5 % (by weight, the balance being ethanol)
- SOLUBILITY:: Soluble in xylene at 70 °C and in normal butanol at 60 °C. A precipitate forms from the n-butanol solution at room temperature on standing.

### EXAMPLE 4

The crosslinking agents of Example 2 and Example 3 and JONCRYL® 500 resin, a product of S.C. Johnson and Son, Inc., Racine, Wisconsin, were cured. The physical properties of the cured films were compared with cured films obtained by using CYMEL® 303 and CYMEL® 1168 crosslinking agents, both products of Cytec Industries Inc., West Paterson, New Jersey. CYMEL® 303 crosslinker is a substantially fully methoxymethylated melamine. CYMEL® 1168 crosslinker is a substantially fully mixed methoxymethylated and isobutoxymethylated melamine. The film properties are summarized in Table 1. The formulations were as follows:
Weight ratio of JONCRYL® 500 Resin/CROSSLINKER: 65/35
Weight percent of para-toluenesulfonic acid (on binder solids): 0.3
Substrate: Electrocoated cold roll steel
Film Thickness: 0.041 - 0.046 mm (1.6 - 1.8 mils)

**TABLE 1**

| FILM PROPERTIES OF COATINGS PREPARED FROM THE NOVEL AMINOPLAST CROSSLINKING AGENTS OF EXAMPLE 2 AND EXAMPLE 3 WITH JONCRYL® 500 RESIN IN CLEAR COATS: A COMPARISON WITH CYMEL® 300 AND CYMEL® 1168 CROSSLINKING AGENTS USED TO PREPARE THE CLEAR COAT | | | | |
|---|---|---|---|---|
| 121 °C/30 MIN. CURE | CYMEL®303 | CYMEL®1168 | EXAM.2 | EXAM.3 |
| TUKON HARDNESS (ASTM D-1474-85) | 10.1 | 7.4 | 9.6 | 10.9 |

| MEK DOUBLE RUBS | | | | |
|---|---|---|---|---|
| TO MAR | 200+ | 200+ | 200+ | 10 |
| TO REMOVE | 200+ | 200+ | 200+ | 200* |

| 149 °C/30 MIN. CURE | | | | |
|---|---|---|---|---|
| TUKON HARDNESS (ASTM D-1474-85) | 11.5 | 8.9 | 10.9 | 12.4 |

| MEK DOUBLE RUBS | | | | |
|---|---|---|---|---|
| TO MAR | 200+ | 200+ | 200+ | 50 |
| TO REMOVE | 200+ | 200+ | 200+ | 200+ |

| | | | | |
|---|---|---|---|---|
| * Film easily scratches off after 200 MEK double rubs. | | | | |

### EXAMPLE 5

The procedure of Example 4 was used to prepare four additional cured films (clear coats) with the exception that both cure catalyst concentration and the JONCRYL® 500 to crosslinker ratios were modified as shown in Table 2. After curing, environmental etch and acid spot tests were carried out.

In the acid spot tests, the cured films are contacted with an acid. After exposure to 38% sulfuric acid at room temperature overnight, no evidence of an acid spot could be detected on films prepared from JONCRYL® 500 and the crosslinker of the invention (Example 2), whereas the film prepared using CYMEL® 1168 had a notable, but slight, haze.

The results of the environmental etch test are summarized in Table 2.

**TABLE 2**

| RESISTANCE PROPERTIES OF THE NOVEL AMINOPLAST CROSSLINKING AGENTS OF EXAMPLE 2 WITH JONCRYL® 500 RESIN IN CLEAR COATS: A COMPARISON WITH CYMEL® 1168 CROSSLINKERS | | | | | |
|---|---|---|---|---|---|
| CROSSLINKERS | JONCRYL®500/CROSSLINKER (RATIO) | CURE CATALYST (para-Toluenesulfonic Acid) (WT. %) | ATTACK RATING* | | |
| | | | H₂SO₄ (50ppm) | HNO₃ (15ppm) | H₂SO₄+HNO₃ (20+20ppm) |
| Example 2 | 65/35 | 0.3 | 5 | 3 | 3 |
| Example 2 | 50/50 | 0.3 | 5 | 1 | 2 |
| Example 2 | 50/50 | 1.0 | 5 | 0 | 1 |
| CYMEL® 1168 | 65/35 | 0.3 | 5 | 3 | 3 |

| | | | | | |
|---|---|---|---|---|---|
| *Attack rating on a scale of 0 to 5. 5 = Greatest Attack 0 = No Visible Attack | | | | | |

### EXAMPLE 6

The procedure of Example 2 is again followed except that 200.o g of butyl formcel are employed such as to react all the NH sites of the melamine moiety. Coatings produced from the resultant aminoplast show excellent properties.

## Claims

1. A composition represented by the formula: 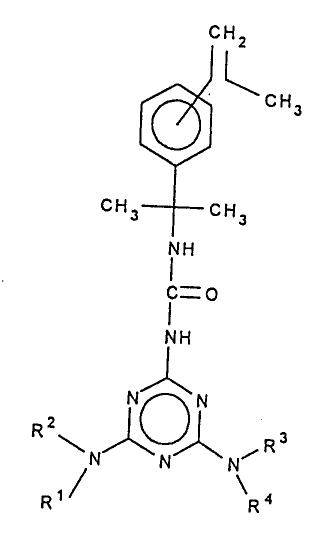 wherein the point of attachment of the isopropenyl group is meta-, para-, or a mixture of thereof, and wherein each of R¹, R², R³, and R⁴ is independently selected from the group consisting of hydrogen, hydroxymethyl, or an alkoxymethyl.

2. The composition of Claim 1 characterized in that at least one of the R¹, R², R³, and R⁴ moieties are independently selected from the group consisting of hydroxymethyl, an alkoxymethyl of 1 to 6 carbon atoms, and mixtures thereof.

3. The composition of Claim 1 represented by the formula:

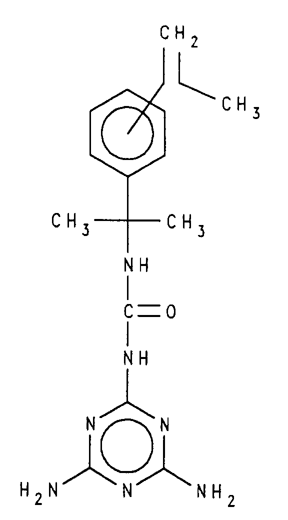

4. The composition of claim 1 represented by the formula: 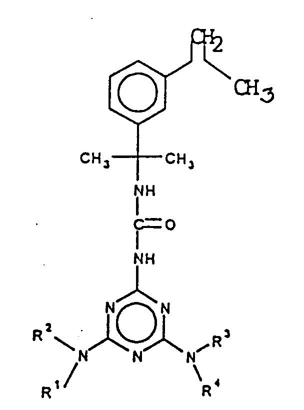 wherein each of R¹, R², R³, and R⁴ is independently selected from the group consisting of hydrogen, hydroxymethyl, or an alkoxymethyl of 1 to 6 carbon atoms.

5. The composition of Claim 4 represented by the formula: 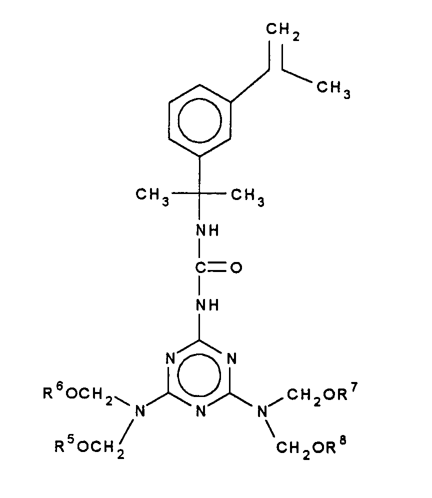 wherein each of R⁵, R⁶, R⁷, and R⁸ is independently selected from the group consisting of hydrogen, methyl, ethyl, 1-propyl, 2-propyl, 1-butyl, 2-butyl, iso-butyl, 1-pentyl, 1-hexyl, and cyclohexyl moieties, and mixtures thereof.

6. The composition of Claim 5 wherein R⁵, R⁶, R⁷ and R⁸ are all hydrogen.

7. A process for preparing a monomeric aminoplast according to claim 1, comprising mono-addition of 2, 4, 6-triamino-1, 3, 5-triazine, and isopropenyl-alpha,alpha-dimethylbenzyl isocyanate, followed by methyloletion and etherification of the product obtained by the mono-additional reaction.

8. A process for preparing an alkoxymethylated aminoplast comprising:
(a) contacting melamine with isopropenyl-alpha,alpha-dimethylbenzyl isocyanate in a solvent having a high dielectric constant, at a temperature and for a length of time sufficient to produce a 1:1 adduct,
(b) contacting the adduct of step (a) with 2 to 20 moles of formaldehyde per mole of adduct to produce a hydroxymethylated adduct, and
(c) contacting said hydroxymethylated adduct of step (b) with 2 to 30 moles of an alcohol per mole of hydroxymethylated adduct at an acidic pH to produce an alkoxymethylated aminoplast.

9. The process of Claim 8 characterized in that the isopropenyl-alpha,alpha-dimethylbenzyl isocyanate in step (a) is meta-isopropenyl-alpha,alpha-dimethylbenzyl isocyanate; para-isopropenyl-alpha,alpha-dimethylbenzyl isocyanate, or a mixture thereof.

10. A curable composition comprising (i) an aminoplast crosslinking agent and (ii) a polyfunctional active hydrogen-containing material, characterized in that the aminoplast crosslinking agent (i) comprises the composition as set forth in any one of claims 1-6 or produced according to the processes set forth in claims 7 or 8.

11. The curable composition of Claim 10 characterized in that the polyfunctional active hydrogen-containing material (ii) comprises at least one class of an active hydrogen functionality selected from the group consisting of hydroxy, carboxy, amido, mercapto, and a group convertible thereto.

12. The curable composition of Claim 11 characterized in that the polyfunctional active hydrogen-containing material (ii) comprises hydroxyfunctional compounds and resins selected from the group consisting of polyhydric alcohols, polyesters, polyurethanes, acrylics, products of condensation of epoxy resins, and mixtures thereof.

13. The curable composition of one of Claims 10 to 12 characterized in that the ratio of the aminoplast crosslinking agent (i) to the polyfunctional active hydrogen-containing material (ii) is from about 3:1 to about 1:40.

14. The curable composition of one of Claims 10 to 13 characterized in that it further comprises from 0.01 to 5.0 weight percent of a cure catalyst.

15. A method of coating a substrate comprising the steps of (I) contacting the substrate with a curable composition containing an aminoplast crosslinking agent (i) and a polyfunctional active hydrogen-containing material (ii), and (II) thereafter curing, characterized in that the aminoplast crosslinking agent (i) comprises the composition as set forth in any one of claims 1-6 or produced according to the processes set forth in claims 7 or 8.

16. The method of Claim 15 characterized in that the curing is carried out at a temperature in the range of from 80 °C to 160 °C.

17. A crosslinked film or object prepared by the method of Claim 15 or Claim 16.

## Patentansprüche

1. Zusammensetzung, dargestellt durch die Formel: 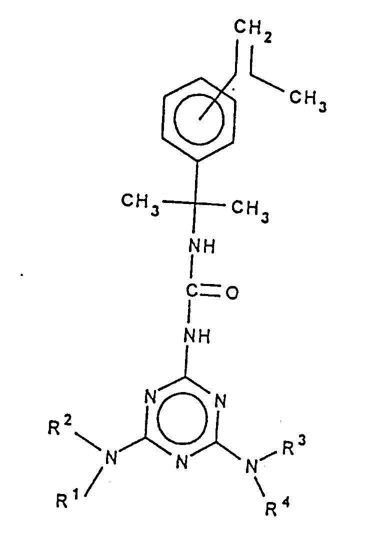 wobei der Befestigungspunkt der Isopropenyl-Gruppe Meta-, Para- oder eine Mischung daraus ist, und wobei jeder aus der Gruppe, umfassend R¹, R², R³ und R⁴, unabhängig aus der Gruppe, bestehend aus Wasserstoff, Hydroxymethyl oder ein Alkoxymethyl, ausgewählt wird.

2. Zusammensetzung nach Anspruch 1, dadurch gekennzeichnet, daß zumindest einer der R¹, R², R³ und R⁴-Substituenten unabhängig aus der Gruppe, bestehend aus Hydroxymethyl, einem Alkoxymethyl aus 1 bis 6 Kohlenstoffatomen und Mischungen daraus, ausgewählt wird.

3. Zusammensetzung nach Anspruch 1, dargestellt durch die Formel:

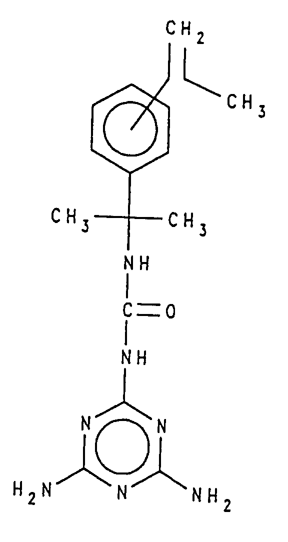

4. Zusammensetzung nach Anspruch 1, dargestellt durch die Formel: 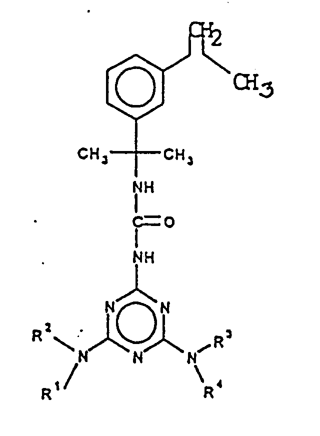 wobei jeder aus der Gruppe, umfassend R¹, R², R³ und R⁴, unabhängig aus der Gruppe, bestehend aus Wasserstoff, Hydroxymethyl oder ein Alkoxymethyl aus 1 bis 6 Kohlenstoffatomen, ausgewählt wird.

5. Zusammensetzung nach Anspruch 4, dargestellt durch die Formel: 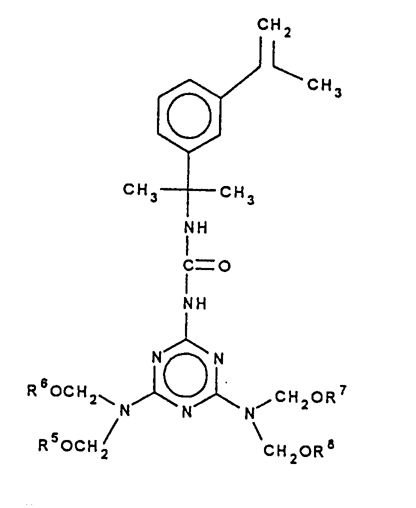 wobei jeder aus der Gruppe, umfassend R⁵, R⁶, R⁷ und R⁸, unabhängig aus der Gruppe, bestehend aus Wasserstoff-, Methyl-, Ethyl-, 1-Propyl-, 2-Propyl-, 1-Butyl-, 2-Butyl-, Isobutyl-, 1-Pentyl-, 1-Hexyl- und Cyclohexyl-Substituenten und Mischungen daraus, ausgewählt wird.

6. Zusammensetzung nach Anspruch 5, wobei R⁵, R⁶, R⁷ und R⁸ allesamt Wasserstoff sind.

7. Verfahren zum Zubereiten eines monomeren Aminoplasts nach Anspruch 1, umfassend die Mono-Addition von 2,4,6-Triamino-1,3,5-triazin und Isopropenyl-alpha,alpha-dimethylbenzylisocyanat, gefolgt von Methylolierung und Etherifizierung des durch die Mono-Additions-Reaktion gewonnenen Produktes.

8. Verfahren zum Zubereiten eines alkoxymethylierten Aminoplasts, umfassend:
(a) Zusammenführen von Melamin mit Isopropenyl-alpha,alpha-dimethylbenzylisocyanat in einem Lösemittel mit einer hohen Dielektrizitätskonstante bei einer Temperatur und während eines Zeitraumes, welche ausreichen, um ein 1:1-Addukt herzustellen,
(b) Zusammenführen des Addukts aus Schritt (a) mit 2 bis 20 Mol Formaldehyd je Mol Addukt, um ein hydroxymethyliertes Addukt zu erzeugen, und
(c) Zusammenführen des hydroxymethylierten Addukts aus Schritt (b) mit 2 bis 30 Mol eines Alkohols je Mol hydroxymethylierten Addukts bei saurem pH-Wert, um ein alkoxymethyliertes Aminoplast herzustellen.

9. Verfahren nach Anspruch 8, dadurch gekennzeichnet, daß das Isopropenyl-alpha,alpha-dimethylbenzylisocyanat in Schritt (a) Meta-isopropenyl-alpha,alpha-dimethylbenzylisocyanat; Para-isopropenyl-alpha,alpha-dimethylbenzylisocyanat oder eine Mischung daraus ist.

10. Härtbare Zusammensetzung, umfassend (i) ein Aminoplast-Vernetzungsmittel und (ii) ein polyfunktionelles, aktiven Wasserstoff enthaltendes Material, dadurch gekennzeichnet, daß das Aminoplast-Vernetzungsmittel (i) die Zusammensetzung umfaßt, welche in einem beliebigen der Ansprüche 1 - 6 dargelegt oder gemäß den Verfahren nach Anspruch 7 oder 8 hergestellt wurde.

11. Härtbare Zusammensetzung nach Anspruch 10, dadurch gekennzeichnet, daß das polyfunktionelle, aktiven Wasserstoff enthaltende Material (ii) zumindest eine Klasse einer aktiven Wasserstoff-Funktionalität umfaßt, ausgewählt aus der Gruppe, bestehend aus Hydroxy-, Carboxy-, Amid-, Mercapto- und einer dazu umwandelbaren Gruppe.

12. Härtbare Zusammensetzung nach Anspruch 11, dadurch gekennzeichnet, daß das polyfunktionelle, aktiven Wasserstoff enthaltende Material (ii) hydroxyfunktionelle Verbindungen und Harze umfaßt, welche aus der Gruppe, bestehend aus mehrere Hydroxylgruppen enthaltenden Alkoholen, Polyestern, Polyurethanen, Acrylharzderivaten, Kondensationsprodukten von Epoxidharzen und Mischungen daraus, ausgewählt werden.

13. Härtbare Zusammensetzung nach einem der Ansprüche 10 bis 12, dadurch gekennzeichnet, daß das Verhältnis des Aminoplast-Vernetzungsmittels (i) zum polyfunktionellen, aktiven Wasserstoff enthaltenden Material (ii) von ungefähr 3:1 bis ungefähr 1:40 beträgt.

14. Härtbare Zusammensetzung nach einem der Ansprüche 10 bis 13, dadurch gekennzeichnet, daß sie des weiteren 0,01 bis 5,0 Gewichtsprozent eines Härtungskatalysators umfaßt.

15. Verfahren des Beschichtens eines Substrats, umfassend die Schritte des (I) Zusammenbringens des Substrats mit einer härtbaren Zusammensetzung, welche ein Aminoplast-Vernetzungsmittel (i) und ein polyfunktionelles, aktiven Wasserstoff enthaltendes Material (ii) enthält, und des (II) nachfolgenden Härtens, dadurch gekennzeichnet, daß das Aminoplast-Vernetzungsmittel (i) die Zusammensetzung umfaßt, welche in einem beliebigen der Ansprüche 1 - 6 dargelegt oder gemäß den Verfahren nach Anspruch 7 oder 8 hergestellt wird.

16. Verfahren nach Anspruch 15, dadurch gekennzeichnet, daß das Härten bei einer Temperatur im Bereich von 80 °C bis 160 °C durchgeführt wird.

17. Vernetzte Folie oder vernetzter Gegenstand, welche(r) durch das Verfahren nach Anspruch 5 oder 6 hergestellt wird.

## Revendications

1. Composition représentée par la formule : 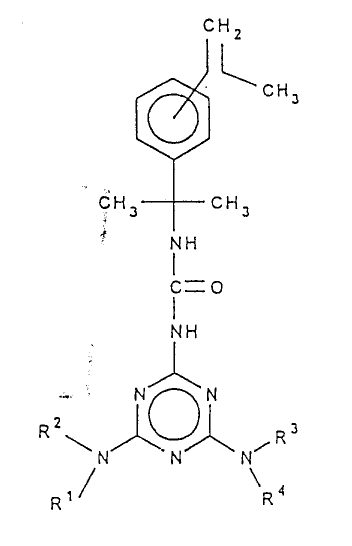 dans laquelle le point de fixation du groupe isopropényle est en *méta* ou *para*, ou un mélange des deux, et dans laquelle chacun de R¹, R², R³ et R⁴ est choisi indépendamment dans la classe formée par un atome d'hydrogène, un groupe hydroxyméthyle, un groupe alcoxyméthyle.

2. Composition de la revendication 1, caractérisée en ce qu'au moins l'un des fragments R¹, R², R³ et R⁴ est choisi indépendamment dans la classe formée par un groupe hydroxyméthyle, un groupe alcoxyméthyle de 1 à 6 atomes de carbone, et leurs mélanges.

3. Composition de la revendication 1, représentée par la formule :

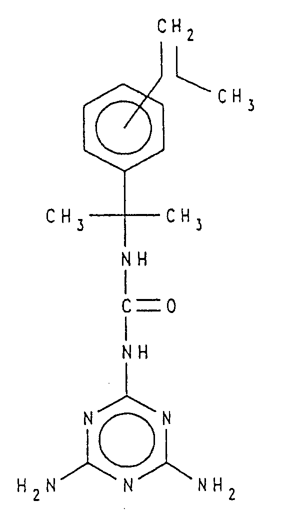

4. Composition de la revendication 1, représentée par la formule : 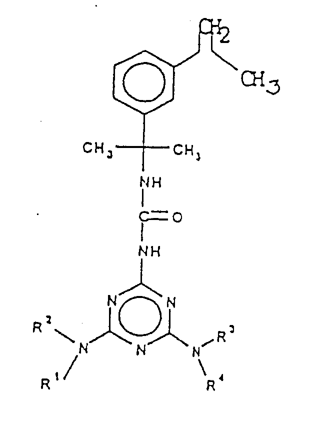 dans laquelle chacun de R¹, R², R³ et R⁴ est choisi indépendamment dans la classe formée par un atome d'hydrogène, un groupe hydroxyméthyle ou un groupe alcoxyméthyle de 1 à 6 atomes de carbone.

5. Composition de la revendication 4, représentée par la formule : 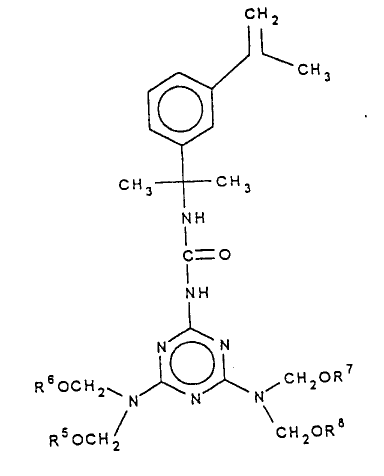 dans laquelle chacun de R⁵, R⁶, R⁷ et R⁸ est choisi indépendamment dans la classe formée par l'hydrogène, les groupes méthyle, éthyle, 1-propyle, 2-propyle, 1-butyle, 2-butyle, isobutyle, 1-pentyle, 1-hexyle et cyclohexyle, et leurs mélanges.

6. Composition de la revendication 5, dans laquelle R⁵, R⁶, R⁷ et R⁸ sont tous des atomes d'hydrogène.

7. Procédé de préparation d'un aminoplaste monomère selon la revendication 1, comprenant une mono-addition de 2,4,6-triamino-1,3,5-triazine et d'isocyanate d'isopropényl-alpha,alpha-diméthylbenzyle, suivie par une méthylolation et une éthérification du produit obtenu par la réaction de monoaddition.

8. Procédé de préparation d'un aminoplaste alcoxyméthylé, comprenant :
(a) la mise en contact de mélamine avec un isocyanate d'isopropényl-alpha,alpha-diméthylbenzyle dans un solvant ayant une haute constante diélectrique, à une température et pendant une durée suffisantes pour produire un produit d'addition à 1:1,
(b) la mise en contact du produit d'addition de l'étape (a) avec 2 à 20 moles de formaldéhyde par mole de produit d'addition pour produire un produit d'addition hydroxyméthylé, et
(c) la mise en contact dudit produit d'addition hydroxyméthylé de l'étape (b) avec 2 à 30 moles d'un alcool par mole de produit d'addition hydroxyméthylé à un pH acide pour produire un aminoplaste alcoxyméthylé.

9. Procédé de la revendication 8, caractérisé en ce que l'isocyanate d'isopropényl-alpha,alpha-diméthylbenzyle de l'étape (a) est l'isocyanate de *méta*-isopropényl-alpha, alpha-diméthylbenzyle, l'isocyanate de *para*-isopropényl-alpha,alpha-diméthylbenzyle, ou un mélange d'entre eux.

10. Composition durcissable comprenant (i) un agent de réticulation du type aminoplaste et (ii) une matière polyfonctionnelle contenant de l'hydrogène actif, caractérisée en ce que l'agent de réticulation du type aminoplaste (i) comprend la composition telle que définie dans l'une quelconque des revendications 1 à 6 ou produite selon les procédés définis dans les revendications 7 ou 8.

11. Composition durcissable de la revendication 10, caractérisée en ce que la matière polyfonctionnelle contenant de l'hydrogène actif (ii) comprend au moins une classe de fonctionnalité à hydrogène actif choisie parmi les groupes hydroxyle, carboxyle, amido, mercapto et un groupe convertible en l'un d'eux.

12. Composition durcissable de la revendication 11, caractérisé en ce que la matière polyfonctionnelle contenant de l'hydrogène actif (ii) comprend des composés et des résines à fonctionnalité hydroxyle choisis dans la classe formée par les polyols, les polyesters, les polyuréthannes, les composés acryliques, les produits de condensation de résines époxy, et leurs mélanges.

13. Composition durcissable de l'une des revendications 10 à 12, caractérisée en ce que le rapport de l'agent de réticulation du type aminoplaste (i) à la matière polyfonctionnelle contenant de l'hydrogène actif (ii) est d'environ 3:1 à environ 1:40.

14. Composition durcissable de l'une des revendications 10 à 13, caractérisée en ce qu'elle contient, de plus, 0,01 à 5,0 pour cent en poids d'un catalyseur de durcissement.

15. Procédé pour revêtir un substrat, comprenant les étapes de (I) mise en contact du substrat avec une composition durcissable contenant un agent de réticulation du type aminoplaste (i) et une matière polyfonctionnelle contenant de l'hydrogène actif (ii), puis (II) durcissement, caractérisé en ce que l'agent de réticulation du type aminoplaste (i) comprend la composition définie dans l'une quelconque des revendications 1 à 6 ou produite selon les procédés définis dans les revendications 7 ou 8.

16. Procédé de la revendication 15, caractérisé en ce que le durcissement est effectué à une température comprise dans l'intervalle de 80°C à 160%.

17. Film ou article réticulé, préparé par le procédé de la revendication 15 ou de la revendication 16.
